# EUROPÄISCHE PATENTSCHRIFT

(11) **EP 0 489 280 B1**
(45) Veröffentlichungstag und Bekanntmachung des Hinweises auf die Patenterteilung: **19.07.1995**
(21) Anmeldenummer: 91119314.2
(22) Anmeldetag: 13.11.1991
(51) Int. Cl.: A61M 16/00

(54) **Fördereinheit für ein Beatmungsgerät**
Gas delivery unit for a respiratory ventilator
Unité d'alimentation pour un respirateur

(30) Priorität: 03.12.1990 DE 4038499
(43) Veröffentlichungstag der Anmeldung: 10.06.1992
(73) Patentinhaber: DRÄGERWERK AKTIENGESELLSCHAFT, 23558 Lübeck (DE)
(72) Erfinder: Leyer, Thomas, W-2400 Lübeck (DE); Türker, Ahmet, W-2400 Lübeck (DE)

(56) Entgegenhaltungen:
- US-A- 4 010 761

## Beschreibung

Die Erfindung betrifft eine Fördereinheit für ein Beatmungsgerät mit einer Kolben-Zylindereinheit als Antriebvorrichtung, bei welcher ein Kolben gegenüber einem Zylinder durch eine topfförmige Beutelrollmembran und eine symmetrisch zur Beutelrollmembran angeordnete Rollmembran abgedichtet ist, mit einer von der Beutelrollmembran umschlossenen Arbeitskammer, welche eine auf einer Stirnseite des Zylinders angebrachte Anschlußplatte als feststehendes Kammerteil und eine Stirnplatte des Kolbens als bewegliches Kammerteil besitzt, wobei die Beutelrollmembran mit ihrer Bodenfläche an der Stirnplatte des Kolbens und mit ihrem offenen Ende an der Stirnseite des Zylinders anliegt, mit einem bei der Kolbenbewegung mitlaufenden Innenraum zwischen den Rollmembranen, der einerseits von einer Kolbenwand des Kolbens und einer Zylinderwand des Zylinders und andererseits von Falten der Rollmembranen umschlossen ist und auf einen gegenüber der Arbeitskammer abgesenkten Druck eingestellt ist

Aus der US 4,010,761 ist eine Fördereinheit für ein Beatmungsgerät bekannt, welche eine Kolben-Zylinder-Einheit als Antriebsvorrichtung mit zwei symmetrisch zueinander angeordneten Rollmembranen zur Abdichtung des Kolbens gegenüber dem Zylinder besitzt.

Eine der Rollmembranen ist als topfförmige Beutelrollmembran ausgeführt und mit einer Anschlußplatte an einer der Stirnseiten des Zylinders befestigt. Die Beutelrollmembran trennt aus dem Zylinderinhalt eine volumenveränderbare Arbeitskammer ab. Der Boden der Beutelrollmembran liegt auf dem den Atemgasförderhub ausführenden Kolben der Kolben-Zylinder-Einheit auf.

Nachteilig bei der bekannten Fördereinheit ist, daß zur Reinigung von atemgasführenden Teilen die Beutelrollmembran und die Anschlußplatte einzeln aus der Antriebsvorrichtung entfernt werden müssen.

Eine Fördereinheit für ein Beatmungsgerät mit einer Kolben-Zylindereinheit als Antriebsvorrichtung ist aus der DE-A1 38 17 091 bekanntgeworden. Der in einem Zylinder geführte Kolben ist gegenüber dem Zylinder durch ein symmetrisch zueinander angeordnetes Rollmembranpaar abgedichtet, welches den Zylinderinhalt in eine Vorkammer und eine Arbeitskammer unterteilt.

Um den Verschleiß der Rollmembranen durch ein sauberes Abrollen der Falten zu verringern, wird der von den Rollmembranen gemeinsam eingeschlossene Innenraum unter einem Druck gehalten, der höher ist als der Druck sowohl in der Vorkammer als auch in der Arbeitskammer und der auf den konkaven Innenseiten der Falten der Rollmembran ansteht, wodurch während des gesamten Atemhubs für eine gleichbleibende Ausbildung der Falten und ihr glattes Anliegen an den Wänden von Kolben und Zylinder gesorgt ist. Sind hingegen die konvexen Außenseiten der Falten einander zugekehrt, ist der Innenraum auf einem Unterdruck gegenüber den benachbarten Kammern gehalten. Eine Dichtigkeitskontrolle der Rollmembran während des Betriebes ist dadurch möglich, daß der Druck im Innenraum überwacht wird und so Leckagen unverzüglich erkennbar sind. Während der Beatmung wird der Kolben über eine Antriebseinheit oszillatorisch bewegt und dabei das während des Arbeitshubes aus der Arbeitskammer verdrängte Atemgas über einen an der Anschlußplatte angebrachten Anschlußstutzen zu einem Atemsystem befördert. Hierbei ist eine genaue Dosierung sowohl von kleinen als auch großen Atemvolumina möglich, da die Fördereinheit, bedingt durch die starre Kolben- und Zylinderwand, eine geringe Compliance hat und im oberen Totpunkt des Kolbens das gesamte Gasvolumen aus der Arbeitskammer ausgedrückt ist. Die Compliance der Rollmembranen ist von untergeordneter Bedeutung, da diese nur im Randbereich im Spalt zwischen Kolben und Zylinder wirksam ist.

Bei der bekannten Kolben-Zylindereinheit ist es von Nachteil, daß während des Betriebs die gesamte Arbeitskammer, die begrenzt ist durch die Stirnplatte des Kolbens, die Innenseite der Anschlußplatte und die Zylinderwand, vom Atemgas durchspült wird und deswegen die gesamte Kolben-Zylindereinheit bei der Geräteaufbereitung gereinigt und desinfiziert werden muß. Sollen bei einem Patientenwechsel die atemgasführenden Teile des Beatmungsgerätes ausgetauscht werden, muß immer eine komplette Kolben-Zylindereinheit vorgehalten werden, obwohl nur die Arbeitskammer mit dem Atemgas in Berührung gekommen ist. Dies erschwert die Handhabung im klinischen Routinebetrieb.

Aus der DE-B2 26 47 343 ist ein Beatmungsgerät bekannt mit einer als Faltenbalg ausgeführten volumenveränderbaren Kammer, die von einem Antriebsmittel in oszillatorische Bewegung versetzt wird, und einschubartig mit dem Beatmungsgerät gekoppelt werden kann. Der Faltenbalg ist über Befestigungsmittel, die hier als Magnetkupplungen ausgeführt sind, mit dem beweglichen Teil des Antriebs verbunden. Durch die einschubartige Konstruktion können die mit dem Atemgas in Berührung kommenden Teile, wie z.B. der Faltenbalg, entnommen und losgelöst vom Beatmungsgerät sterilisiert werden.

Bei dem bekannten Beatmungsgerät ist es von Nachteil, daß mit einer einzigen Faltenbalg-Größe als volumenveränderbare Arbeitskammer nur eine ungenaue Dosierung des Atemhubvolumens möglich ist, da der Faltenbalg aufgrund seiner Eigenelastizität eine hohe Compliance besitzt, wodurch speziell die Dosierung von kleinen Atemhubvolumina - wie sie zur Beatmung von Kleinkindern notwendig sind - sehr ungenau wird. Es ist zwar von anderen Beatmungsgeräten bekannt, in diesen Fällen den Faltenbalg mit großem Arbeitskammer-Volumen gegen eine Ausführung mit kleinerem Arbeitskammer-Volumen auszutauschen, hierdurch wird jedoch die Handhabung erschwert, z.B. wenn während einer Beatmung aus therapeutischen Gründen kurzfristig ein höheres Atemhubvolumen appliziert werden muß. Außerdem sind besondere Überwachungseinrichtungen notwendig, die dem Anwender anzeigen, welcher Faltenbalg gerade im Beatmungsgerät eingesetzt ist.

Der Erfindung liegt die Aufgabe zugrunde, eine Fördereinheit für ein Beatmungsgerät derart zu verbessern, daß bei genauer Dosierung sowohl von großen und von kleinen Atemhubvolumina mit dem gleichen Arbeitskammer-Volumen, die atemgasführenden Teile einfach in die Antriebsvorrichtung einsetzbar und aus dieser wieder entnehmbar sind.

Die Lösung der Aufgabe erfolgt dadurch, daß die Anschlußplatte ein Befestigungsmittel aufweist, in welches das offene Ende der Beutelrollmembran einknöpfbar ist, so daß die Beutelrollmembran, zusammen mit der Anschlußplatte, einschubartig in den Zylinder einsetzbar ist.

Der Vorteil der Erfindung besteht im wesentlichen darin, daß bei der Kolben-Zylindereinheit mit zwei Rollmembranen eine Rollmembran als topfförmige, mit dem Anschlußstück verbundene Beutelrollmembran ausgeführt ist, welche als Arbeitskammer einschubartig in die Antriebsvorrichtung einsetzbar ist und der Innenraum zwischen den Rollmembranen an eine Vakuumquelle angeschlossen und auf einem Druckniveau unterhalb des Druckes in der Arbeitskammer gehalten ist. Durch den Unterdruck zwischen dem Innenraum und der Arbeitskammer legt sich die Beutelrollmembran an die Zylinderwand und die Stirnplatte des Kolbens an, während sich gleichzeitig die Falten zwischen Kolbenwand und Zylinderwand ausbilden.

Die Kombination der Beutelrollmembran mit einer Kolben-Zylindereinheit als Antriebsvorrichtung führt zu der hohen Dosiergenauigkeit wie bei einer Kolben-Zylindereinheit mit fest eingebautem Rollmembranpaar, während gleichzeitig eine leichte Entnahme der atemgasführenden Teile möglich ist, wie bei einer einschubartigen Konstruktion mit Faltenbalg. Mit der erfindungsgemäßen Fördervorrichtung können sowohl große Atemhubvolumina für Erwachsene als auch kleine Atemhubvolumina für Kleinkinder appliziert werden.

Zur Befestigung der Bodenfläche der Beutelrollmembran an der Stirnplatte des Kolbens ist der Unterdruck im Innenraum auf den Haftdruck abgesenkt. Der Haftdruck ist abhängig von dem Durchmesser des Kolbens und des Zylinders und dem Rauminhalt des von der Beutelrollmembran geförderten Atemgases. Versuche haben gezeigt, daß für die praxisrelevanten Atemhubvolumina der Haftdruck etwa um 150 Millibar unter dem in der Arbeitskammer herrschenden mittleren Druck liegt. Die Befestigung der Bodenfläche der Beutelrollmembran auf der Stirnplatte des Kolbens über den Haftdruck im Innenraum bewirkt außerdem eine automatische Zentrierung der Beutelrollmembran innerhalb des Zylinders während des ersten Kolbenhubes. Wurde beispielsweise die Beutelrollmembran verkantet in den Zylinder eingesetzt, legt sie sich unter der Wirkung des Haftdruckes zunächst an die Zylinderwand und die Stirnplatte des Kolbens. Wird der Kolben in den unteren Totpunkt bewegt, bilden sich zwischen Kolben- und Zylinderwand die Falten aus, wodurch die Beutelrollmembran in die Arbeitsposition gezogen wird. Es ist zweckmäßig, als Befestigungsmittel eine Adhäsionsbeschichtung zwischen der Bodenfläche der Beutelrollmembran und der Stirnplatte des Kolbens vorzusehen. Durch die Adhäsionsbeschichtung ist eine Haftung vorhanden unabhängig von dem Druck im Innenraum. Tritt beispielsweise eine Störung innerhalb der Vakuumquelle auf, so daß kein Unterdruck im Innenraum erzeugt werden kann, ist durch die Adhäsionsbeschichtung trotzdem eine Beatmung mit der Kolben-Zylindereinheit möglich. Die Adhäsionsbeschichtung kann ganzflächig auf der Bodenfläche und der Stirnplatte oder segmentartig als punkt- oder kreisringförmige Beschichtung aufgebracht sein.

Vorteilhafte Ausgestaltungen der Erfindung sind in den Unteransprüchen angegeben.

In zweckmäßiger Weise ist das obere Ende der Beutelrollmembran in einen Vorsprung der Anschlußplatte eingeknöpft.

Eine zweckmäßige Ausgestaltung der Erfindung ist, die Anschlußplatte als ein Atemsystem auszuführen, welches zusammen mit der Beutelrollmembran einschubartig als in sich geschlossene Arbeitskammer in den Zylinder einsetzbar ist. Durch die Kombination des Atemsystems mit der Beutelrollmembran können die atemgasführenden Teile des Beatmungsgerätes als Kompaktmodul in den Zylinder eingesetzt oder aus diesem entfernt werden.

Ein Ausführungsbeispiel der Erfindung wird anhand der schematischen Zeichnung dargestellt und im folgenden näher erläutert.

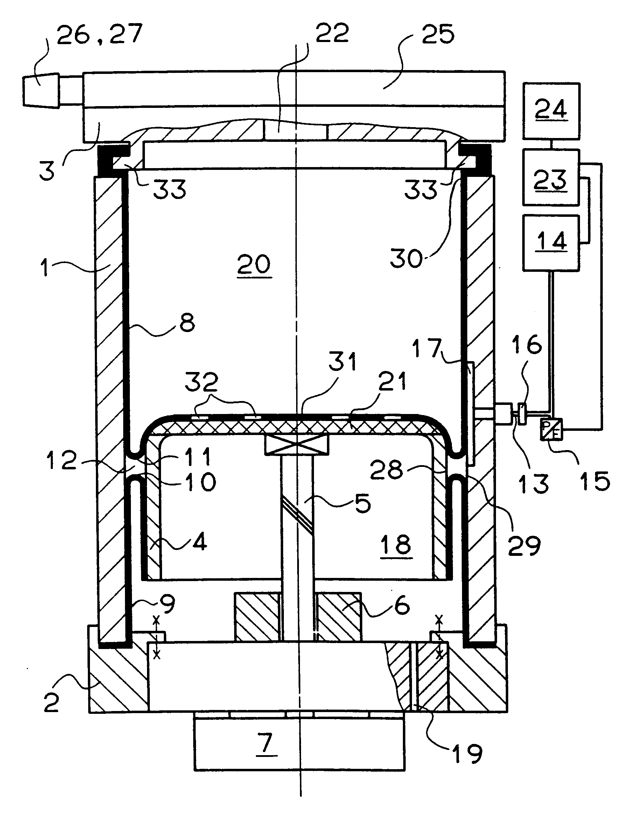

In der einzigen Figur ist ein Zylinder (1) dargestellt, dessen Stirnseiten durch eine Grundplatte (2) und eine Anschlußplatte (3) abgeschlossen sind. Im Innern des Zylinders (1) ist ein Kolben (4) auf einer in einem Axiallager (6) geführten Kolbenstange (5) aufgenommen, die mit einer Antriebsvorrichtung (7) verbunden ist. Die Kolbenwand (28) ist gegenüber dem Zylinder (1) mit einer Rollmembran (9) und einer Beutelrollmembran (8) abgedichtet, wobei das offene Ende (30) der Beutelrollmembran (8) in einen Vorsprung (33) der Anschlußplatte (3) eingeknöpft und die Rollmembran (9) zwischen dem Zylinder (1) und der Grundplatte (2) eingeklemmt ist. Der Vorsprung (33) der Anschlußplatte (3) sitzt durch die umgelegte Beutelrollmembran (8) dichtend auf einer Stirnseite des Zylinders (1). Die konvexen Außenseiten der Falten (10, 11) sind einander zugekehrt, da der von den Rollmembranen (8, 9) eingeschlossene Innenraum (12) über eine Leitung (13) und ein Filter (16) an eine Vakuumquelle (14) angeschlossen ist, die einen Unterdruck von etwa 150 Millibar erzeugt. Die Leitung (13) mündet in einem Verteiler (17), der als Längsnut in der Zylinderwand (29) ausgeführt ist und dazu dient, bei jeder Stellung des Kolbens (4) eine Verbindung zwischen dem Innenraum (12) und der Leitung (13) herzustellen.

Die Rollenmembranen (8, 9) teilen das Innere des Zylinders (1) in eine Vorkammer (18), welche über einen Durchlaß (19) mit der Umgebung verbunden ist, und in eine Arbeitskammer (20), in welcher der durch die Hubbewegung des Kolbens (4) erzeugte Beatmungsdruck herrscht. Der Kolben (4) besitzt an seiner der Arbeitskammer (20) zugewandten Seite eine Stirnplatte (21), an der die Bodenfläche (31) der Beutelrollmembran (8) anliegt. Das während des Arbeitshubes verdrängte Atemgas kann durch den an der Anschlußplatte (3) angebrachten Anschlußstutzen (22) zu einem Atemsystem (25) eines nicht dargestellten Beatmungsgerätes fließen. Das Atemsystem (25) hat Anschlüsse (26, 27) für eine nicht dargestellte Einatemleitung und Ausatemleitung, über die die Atemgasversorgung erfolgt. An die Leitung (13) ist eine Druckmeßeinrichtung (15) angeschlossen, deren Meßsignal an eine Steuereinheit (23) weitergeleitet wird, welche gleichzeitig den von der Vakuumquelle (14) gelieferten Sollwert aufnimmt. Eine Anzeigeeinheit (24) zeigt den aktuellen Zustand des Unterdrucks im Innenraum (12) an und gibt eventuell notwendige Warnsignale ab.

Die Inbetriebnahme der Fördervorrichtung erfolgt in der Weise, daß das Atemsystem (25) mit der Anschlußplatte (3) und der Beutelrollmembran (8) auf den Zylinder (1) gesetzt und die Vakuumquelle (14) eingeschaltet wird. Durch den sich im Innenraum (12) einstellenden Unterdruck legt sich die Beutelrollmembran (8) gegen die Zylinderwand (29) und die Bodenfläche (31) der Beutelrollmembran (8) gegen die Stirnplatte (21) des Kolbens (4). Durch den Unterdruck wird ebenfalls die Anschlußplatte (3) gegen die Stirnseite des Zylinders (1) gepreßt, wobei die an der Trennstelle zwischen Anschlußplatte (3) und Zylinder (1) befindliche Beutelrollmembran (8) für einen gasdichten Verschluß sorgt. Während des ersten Kolbenhubes zentriert sich unter der Wirkung des Unterdrucks die Falte (11) der Beutelrollmembran, zwischen Kolbenwand (28) und Zylinderwand (29), so daß dann ein einwandfreies Abrollen der Falten (10, 11) bei den folgenden Kolbenhüben gewährleistet ist. Für eine gleichmäßige Faltenbildung der Rollmembranen (8, 9) und ein festes Haften der Bodenfläche (31) an der Stirnplatte (21) ist ein Unterdruck oder Haftdruck von etwa 150 Millibar erforderlich. Um bei einem Ausfall der Vakuumquelle (14) ein Haften der Bodenfläche (31) der Beutelrollmembran (8) an der Stirnplatte (21) zu gewährleisten, ist eine punktförmige Adhäsionsbeschichtung (32) an der Berührfläche vorgesehen.

## Patentansprüche

1. Fördereinheit für ein Beatmungsgerät mit einer Kolben-Zylindereinheit als Antriebsvorrichtung, bei welcher ein Kolben (4) gegenüber einem Zylinder (1) durch eine topfförmige Beutelrollmembran (8) und eine symmetrisch zur Beutelrollmembran (8) angeordnete Rollmembran (9) abgedichtet ist, mit einer von der Beutelrollmembran (8) umschlossenen Arbeitskammer (20), welche eine auf einer Stirnseite des Zylinders (1) angebrachte Anschlußplatte (3) als feststehendes Kammerteil und eine Stirnplatte (21) des Kolbens (4) als bewegliches Kammerteil besitzt, wobei die Beutelrollmembran (8) mit ihrer Bodenfläche (31) an der Stirnplatte (21) des Kolbens (4) und mit ihrem offenen Ende (30) an der Stirnseite des Zylinders (1) anliegt, mit einem bei der Kolbenbewegung mitlaufenden Innenraum (12) zwischen den Rollmembranen (8, 9), der einerseits von einer Kolbenwand (28) des Kolbens (4) und einer Zylinderwand (29) des Zylinders (1) und andererseits von Falten (10, 11) der Rollmembranen (8, 9) umschlossen ist und auf einen gegenüber der Arbeitskammer (20) abgesenkten Druck eingestellt ist, dadurch gekennzeichnet, daß die Anschlußplatte (3) ein Befestigungsmittel (33) aufweist, in welches das offene Ende (30) der Beutelrollmembran (8) einknöpfbar ist, so daß die Beutelrollmembran (8), zusammen mit der Anschlußplatte (3), einschubartig in den Zylinder (1) einsetzbar ist.

2. Fördereinheit nach Anspruch 1, dadurch gekennzeichnet, daß das Befestigungsmittel ein Vorsprung (33) ist.

3. Fördereinheit nach Anspruch 1 oder 2, dadurch gekennzeichnet, daß die Anschlußplatte als ein Atemsystem (25) ausgeführt ist.

## Claims

1. Conveying unit for a respiratory apparatus having a piston-cylinder unit as a drive arrangement in which a piston (4) is sealed in respect of a cylinder (1) by means of a pot-shaped bag-like rolling membrane (8) and a rolling membrane (9) which is arranged so as to be symmetrical to the bag-like rolling membrane (8), having a working chamber (20) which is enclosed by the bag-like rolling membrane (8) and has a connecting plate (3), provided on a face of the cylinder (1), as a fixed chamber portion and a front plate (21) of the piston (4) as a movable chamber portion, wherein the bag-like rolling membrane (8) rests with its base surface (31) against the front plate (21) of the piston (4) and with its open end (30) against the face of the cylinder (1), having an interior space (12) between the rolling membranes (8, 9) which follows the movement of the piston and, on the one hand, is enclosed by a piston wall (28) of the piston (4) and a cylinder wall (29) of the cylinder (1) and, on the other hand, is enclosed by folds (10, 11) of the rolling membranes (8, 9) and is adjusted to a pressure which is lowered in respect of the working chamber (20), characterised in that the connecting plate (3) has a securing means (33) into which the open end (30) of the bag-like rolling membrane (8) can be fastened so that the bag-like rolling membrane (8), together with the connecting plate (3), can be inserted into the cylinder (1) in the manner of a plug-in unit.

2. Conveying unit according to claim 1, characterised in that the securing means is a projection (33).

3. Conveying unit according to claim 1 or 2, characterised in that the connecting plate is designed as a respiratory system (25).

## Revendications

1. Unité d'alimentation destinée à un appareil d'assistance respiratoire, pourvue d'une unité piston/cylindre servant de dispositif d'entraînement, dans laquelle un piston (4) est rendu étanche vis-à-vis d'un cylindre (1) par une membrane roulée formant sac (8) en forme de creuset et par une membrane roulée (9) disposée de façon symétrique par rapport à la membrane roulée formant sac (8), pourvue d'une chambre de travail (20) entourée par la membrane roulée formant sac (8), qui présente une plaque de raccordement (3) disposée sur une face frontale du cylindre (1) et servant de partie de chambre fixe, ainsi qu'une plaque frontale (21) du piston (4) servant de partie de chambre mobile, la membrane roulée formant sac (8) venant s'appliquer, par sa surface de fond (31), sur la plaque frontale (21) du piston (4) et, par son extrémité ouverte (30), sur la face frontale du cylindre (1), pourvue d'un espace intérieur (12) suivant le déplacement du piston et existant entre les membranes roulées (8, 9), qui est entouré, d'une part, par une paroi (28) du piston (4) et une paroi (29) du cylindre (1) et, d'autre part, par des plis (10, 11) des membranes roulées (8, 9), et qui est mis à une pression abaissée par rapport à celle de la chambre de travail (20), caractérisée en ce que la plaque de raccordement (3) présente un moyen de fixation (33), dans lequel l'extrémité ouverte (30) de la membrane roulée formant sac (8) est susceptible de s'insérer, de telle sorte que la membrane roulée formant sac (8), avec la plaque de raccordement (3), puisse être introduite dans le cylindre (1) en y étant enfilée.

2. Unité d'alimentation selon la revendication 1, caractérisée en ce que le moyen de fixation est une saillie (33).

3. Unité d'alimentation selon la revendication 1 ou 2, caractérisée en ce que la plaque de raccordement est conformée en système respiratoire (25).
